# EUROPEAN PATENT SPECIFICATION

(11) **EP 0 851 766 B1**
(45) Date of publication and mention of the grant of the patent: **25.05.2005**
(21) Application number: 96935822.5
(22) Date of filing: 16.09.1996
(51) Int. Cl.: G01N 33/53, G01N 33/58

(54) **METHOD FOR ANALYZING THE IMMUNOSUPPRESSANT ACTIVITY OF ION CHANNEL BLOCKERS USING THE MINI-PIG**
METHODE ZUR ANALYSE DER IMMUNSUPPRESSIVEN AKTIVITÄT VON IONENKANAL-BLOCKERN MIT HILFE DES MINI-SCHWEINS
PROCEDE D'ANALYSE DE L'ACTIVITE IMMUNOSUPPRESSIVE DES INHIBITEURS DE CANAUX IONIQUES SUR LA BASE DU MODELE DU PORC MINIATURE

(30) Priority: 19.09.1995 US 603991; 16.02.1996 GB 9603302
(43) Date of publication of application: 08.07.1998
(73) Proprietor: MERCK & CO., INC., Rahway, New Jersey 07065 (US)
(72) Inventor: BLAKE, J., Thomas, Rahway, NJ 07065 (US); FEENEY, William, P., Rahway, NJ 07065 (US); KOO, Gloria, C., Rahway, NJ 07065 (US); TALENTO, Althea, D., Rahway, NJ 07065 (US)
(74) Representative: Horgan, James Michael Frederic
(86) International application number: PCT/US1996/014768
(87) International publication number: WO 1997/010848

(56) References cited:
- KOO GC ET AL: "Voltage-gated potassium channels (Kv1.3) do not regulate the proliferation of mouse T or B cells" CONF: EXPERIMENTAL BIOLOGY 95, PART I, vol. 9, March 1995 (1995-03), page A238 XP000995493 Atlanta, USA
- LIN C. SHIRLEY ET AL: "Voltage-gated potassium channels regulate calcium-dependent pathways involved in human T lymphocyte activation" JOURNAL OF EXPERIMENTAL MEDICINE, vol. 177, 1993, pages 637-645, XP000995416
- IWAMA ET AL.: "Reversing effect of anti-asthmatic drugs on bronchoconstriction induced by antigen challenge and histamine in anesthetized guinea-pigs" JAPANESE JOURNAL OF PHARMACOLOGY, vol. 58, 1992, pages 19-25, XP000995455
- BRAIN, 1989, Vol. 112, MIX et al., "Effect of Ion Channel Blockers on Immune Response and Course of Experimental Allergic Neuritis", pages 1405-1418, XP002945199.
- PERSPECTIVES IN DRUG DISCOVERY AND DESIGN., August 1994, Vol. 2, No. 1, KACZOROWSKI et al., "Lymphocyte Ion Channels as a Target for Immunosuppression", pages 233-248, XP002945200.
- BIOL. ABSTR., Vol. 048, No. 007, 02 June 1996, (New Orleans, La, USA), page A1447, Abstract No. 2573, KOO et al., "Margatoxin, a Blocker of Kv1.3 Channels, Inhibits Immune Responses in Vivo"; & FASEB JOURNAL, Vol. 10, No. 6. AN prev199699032391 XP002903363

## Description

### BACKGROUND OF THE INVENTION

The potassium ion channel is involved with the normal cellular homestasis and its possible association with and derangements relating to a variety of disease states and immune responses.

Diseases having a particular association with such channels include autoimmune diseases and transplant rejections. Autoimmune diseases include rheumatoid arthritis, type-1 diabetes mellitus (insulin dependent), multiple sclerosis, myasthenia gravis, systemic lupus erythematosus, Sjorgran's syndrome, mixed connective tissue disease, experimental allergic encephalomyelitis (EAE), to name a few.

In most cases, it is believed that autoimmune diseases result from auto-reactive cells of the immune system destroying target tissues, either by direct killing or by producing autoantibodies. In the autoimmune diseases studied to date, there seems to emerge a common pattern of abnormal immune cells producing materials that either destroy or retard certain target tissues causing symptoms manifest for that disease state.

Allografts or xenografts may be rejected through either a cell-mediated or a humoral immune reaction of the recipient against antigenic components present on rejection which is the acute lymphocyte-mediated immune reaction against transplantation antigens (host vs. graft reaction).

Current treatment for these responses and diseases remains on an empical level and is based on causing a general immunosuppressant response. Such a therapeutic approach is also fraught with other problems including associated severe side effects. Further, they serve only to retard the natural progression of these autoimmune diseases.

Several classes of potassium channels are involved in maintaining membrane potential and regulating cell volume in diverse cell types, as well as modulating electrical excitability in the nervous system. [R.C. Lewis and M.D. Cahalan "Potassium and Calcium Channels in Lymphocytes" Ann. Rev. Immunol. *13*, (1995) 623-653 and "Lymphocyte Ion Channels as a Target for Immunosuppression" G.J. Kaczorowski and G.C. Koo Perspectives in Drug Discovery and Design 2, (1994) 233-248.] Potassium channels have been shown to control the repolarization phase of action potentials and the pattern of firing neurons and other cells. Potassium currents have been shown to be more diverse than sodium or calcium currents, and also play a central role in determining the way a cell responds to an external stimulus. For instance, the rate of adaptation or delay with which a neuron responds to synaptic input is strongly determined by the presence of different classes of potassium channnels.

Attention has been focused on the K⁺ ion channel itself. Two types of ion channel types, classified pharmacologically and electrophysiologically, have been identified, the voltage activated potassium channels (Kv) and the calcium activated K⁺ channels (K_{Ca}). T cells in the peripheral lymphoid tissues for present purposes are characterized into relevant types: CD₄⁺CD₈⁻, CD₄⁻CD₈⁺, CD₄⁻CD₈⁻. These cells express some or all of these potassium ion channels. In the normal immune response reflecting induction of activity, such as with mitogens, a single ion channel type is increased upwards of ten fold in the cells that are activated. Thus, normal T cells, when stimulated by mitogens, show a normal immune response elevation in the number of these ion channels.

One of these types of K⁺ ion channels, Kv1.3, determines membrane potential in non-actived human T-cells. It has been determined that blockade of this K⁺ ion channel is sufficient to cause depolarization and prevent activation of mitogen. [Lymphocyte Ion Channels as a Target for Immunosuppression" G.J. Kaczorowski and G.C. Koo Perspectives in Drug Discovery and Design 2, (1994) 233-248.]

Prior to the discovery of the mini-pig as an animal model for testing Kᵥ1.3 blockers in the mixed lymphocyte reaction, several other animal models were studied, including the mouse, rat, rabbit and guinea pig. However, the mixed lymphocyte reaction is not inhibited by Margatoxin (MgTX), a specific peptide inhibitor of Kv 1.3 channels in these animal models. [Leonard et al. Selective blockers of voltage gated K⁺ channels depolarized human T lymphocytes: mechanism of the antiproliferatice effect of Charybdotoxin. Proc. Natl. Acad. Sci. USA *89*: 10094-10098.]

Mini-swine was recently selected to validate the concept that blocking the voltage activated K+ channels (Kv1.3) is a target for immunomodulation. The scorpion toxin, Margatoxin (MgTX) has been shown to block the Kv1.3 channels of the pig T cells in electrophysiological analyses (unpublished communication from Reid Leonard). Margatoxin has also been found to inhibit the mixed lymphocyte reaction in the pig, similar to its effect in human mononuclear cells (MNC). PMA/ionomycin (ION) induced proliferation of pig mononuclear cells was equally inhibited. Therefore in vitro assays indicate that pig Kv1.3 channels are comparable to human Kv 1.3 channels in their responses to margatoxin.

T cell mediated delayed type hypersensitivity (DTH) response in the pig was then chosen to test the effect of ion channel blockers *in vivo.* Margatoxin would be used in DTH, for the lack of specific compound that inhibits the Kv1.3 channels. Therefore, pharmacokinetic (PK) study of margatoxin was conducted in the mini-swine. In addition to determining the concentration of margatoxin in the plasma, we developed an *ex vivo* assay to assess the response of the mononuclear cells to the induction of PMA and ionomycin (ION). This assay is currently used to monitor the *in vivo* biological effect of margatoxin.

### SUMMARY OF THE INVENTION

This invention relates to methods for analyzing the immunosuppressant activity of Kv1.3 ion channel blockers using the pig as the animal model. This invention relates to a method for analyzing an *ex vivo* effect of a Kv1.3 ion channel blocker administered *in vivo* to a test pig wherein the analysis comprises the steps of:
(a) immunizing the test pig and a control pig with an antigen;
(b) measuring the immune response of the test and the control pig;
(c) administering the Kv1.3 ion channel blocker to the test pig;
(d) administering the vehicle to the control pig;
(e) measuring the immune response of the test and the control pig;
(f) challenging the test and the control pig with the antigen;
(g) measuring the immune response of the test pig relative to the immune response of the control pig; and
(h) measuring the antigen response of the test and the control pig relative to the antigen response of the control pig.

### BRIEF DESCRIPTION OF THE FIGURES

Figure 1.
   Pharmacokinetics of Margatoxin: Inhibition of *ex vivo* PMA/ionomycin-induced proliferation by infusion of 2 µg/kg of the immunosuppressant, Margatoxin.
Figure 2.
   Inhibition of the delayed type hypersensitivity (DTH) response to Tuberculin (PPD) in the mini-pig.

### DETAILED DESCRIPTION OF THE INVENTION

This invention relates to a method for analyzing an *ex vivo* effect of a Kv1.3 ion channel blocker administered *in vivo* to a test pig wherein the analysis comprises the steps of:
(a) immunizing the test pig and a control pig with an antigen;
(b) measuring the immune response of the test and the control pig;
(c) administering the Kv1.3 ion channel blocker to the test pig;
(d) administering the vehicle to the control pig;
(e) measuring the immune response of the test and the control pig;
(f) challenging the test and the control pig with the antigen;
(g) measuring the immune response of the test pig relative to the immune response of the control pig; and
(h) measuring the antigen response of the test and the control pig relative to the antigen response of the control pig.

An embodiment of the invention is the method as recited above wherein the measurement of the immune response comprises the steps of:
(a) collecting whole heparinized blood from the pig;
(b) diluting the whole heparinized blood with an equal volume of RPMI;
(c) centrifuging the RPMI-diluted blood solution over ficoll-hypaque;
(d) isolating the mononuclear cell fraction from the centrifuged RPMI-diluted blood solution;
(e) adjusting the concentration of the mononuclear cell fraction to about 2-million cells per mL;
(f) reacting 100µL of a solution of PMA at 0.1 to 0.5 ng/mL and ionomycin at 200 ng/mL to the microtiter wells with 100 µL of the mononuclear cells;
(g) incubating the PMA-ionomycin reaction for about 24 hours;
(h) incubating the incubated PMA-ionomycin reaction for an additional 24 hours with ³H-thymidine;
(i) harvesting the mononuclear cells after incubation with ³H-thymidine;
(j) counting the ³H-thymidine incorporation into the mononuclear cells.

The above method is used to measure the immunosuppressant activity of the ion channel blocker in mononuclear cells isolated from blood at various time intervals during the experiment, relative to a control. The samples were prepared without the washing step typically employed when isolating the mononuclear cells from whole heparinized blood, which allowed for rapid detection of the immune responsiveness, after *in vivo* administration of an ion channel blocker.

This invention also relates to a method for analyzing an *ex vivo* effect of a Kv1.3 ion channel blocker administered *in vivo* to a test pig wherein the analysis comprises the steps of:
(a) immunizing the test pig and a control pig with an antigen;
(b) measuring the immune response of the test and the control pig comprising the steps of:
   (i) collecting a sample of whole heparinized blood from the test and control pigs;
   (ii) diluting the test and control samples of whole heparinized blood with an equal volume of RPMI;
   (iii) centrifuging the test and control samples of RPMI-diluted blood solution over ficoll-hypaque;
   (iv) isolating the test and control samples of the mononuclear cell fraction from the centrifuged RPMI-diluted blood solution; .
   (v) adjusting the concentration of the test and control samples of the mononuclear cell fraction to about 2-million cells per mL;
   (vi) reacting 100µL of a solution of PMA at 0.1 to 0.5 ng/mL and ionomycin at 200 ng/mL to the microtiter wells with 100 µL of the test and control samples of the mononuclear cells;
   (vii) incubating the test and control samples of the PMA-ionomycin reaction for about 24 hours;
   (viii) incubating the test and control samples of the incubated PMA-ionomycin reaction for an additional 24 hours with ³H -thymidine;
   (ix) harvesting the test and control samples of the mononuclear cells after incubation with ³H-thymidine;
   (x) counting the ³H-thymidine incorporation into the test and control samples of the mononuclear cells to establish a baseline;
(c) administering into a vein of the test pig a solution of the Kv1.3 ion channel blocker at a daily dose of 2 µg/kg to 100 mg/kg using an infusion pump dosing at a rate of 2.0 to 12 mL/hour;
(d) measuring the immune response of the dosed-test-pig as recited above in step (b);
(e) challenging the test and control pigs with the antigen;
(f) measuring the immune response of the antigen-challenged-test-pig and control pig as recited above in step (b);
(g) measuring the antigen response of the test pig and the control pig.

An embodiment of the invention is a method for analyzing an *ex vivo* effect of a Kv1.3 ion channel blocker administered *in vivo* to a pig wherein for said analysis the pig is immunized with Bacillus-Calmette-Guerin and later challenged with purified protein derivative-tuberculin (PPD).

The term pig used in the instant invention includes the mini-pig and the micro-pig, the pigs ranging in weight from about 2kg to about 15kg. The preferred animal model being the Yucatan mini-pig or the Yucatan micro-pig. Other strains of mini-pigs are Hanford, NIH and Minnesota varieties. The Yucatan mini-pigs and Yucatan micro-pigs were obtained from Charles River, Windham, ME.

The ion channel blocker is administered to the pig either intravenously, orally, intramuscularly or subcutaneously. The preferred route of adminstration is selected from intravenous or oral.

The daily dose of the ion channel blocker is between about 2 µg/kg to about 100 mg/kg. The preferred daily dose of an ion channel blocker is about 0.1 mg/kg to about 100 mg/kg when administered orally and about 2 µg/kg to about 15 mg/kg when administered intravenously. Margatoxin has been found to be effective in a range from about 4 µg/kg to about 8 µg/kg.

An ambulatory infusion pump (Pharmacia-Delta) or a portable infusion pump was used to deliver the ion channel blocker, e.g. margatoxin, at a pre-programmed rate to the pig. The reservoir within the pump were filled daily with a solution of the ion channel blocker, when the compound was delivered every day. The chosen dose of ion channel blocker was diluted in 30 ml of 1:1 saline and PBS, containing the 5% autologous serum. The pigs were instrumented with catheters at the jugular vein and artery, several days before the assay. At the time of treatment, the ambulatory infusion pump was mounted in the pocket of a custom-made vest, made of polyester net material (Fabric Expression, Seattle, WA) or the pig is restrained in a sling during the duration of the study and the intravenous catheter is connected to a portable infusion pump. The margatoxin solution was delivered by an infusion pump, at a rate of about 2 to about 12 ml/hr. The margatoxin solution is infused three times a day dosing over a four hour period and was delivered either on the eight days following the immunization or on the day of the challenge. The pigs were then immunized with 1 mg of Bacillus-Calmette-Guerin (Connaugh) intradermally and subcutaneously at about 4 to about 6 different sites on the rump. On the day (7-10 days post immunization) of challenge, 0.1 ml of tuberculin (PPD) (25 Units, Connaugh) was injected intradermally, when the pig was sedated with Telazol and /or isoflurane. See the timeline set forth below. Induration was measured 48 hrs. later. Blood was collected at various times during and after infusion for biochemical determinations and biological assays, as described below.

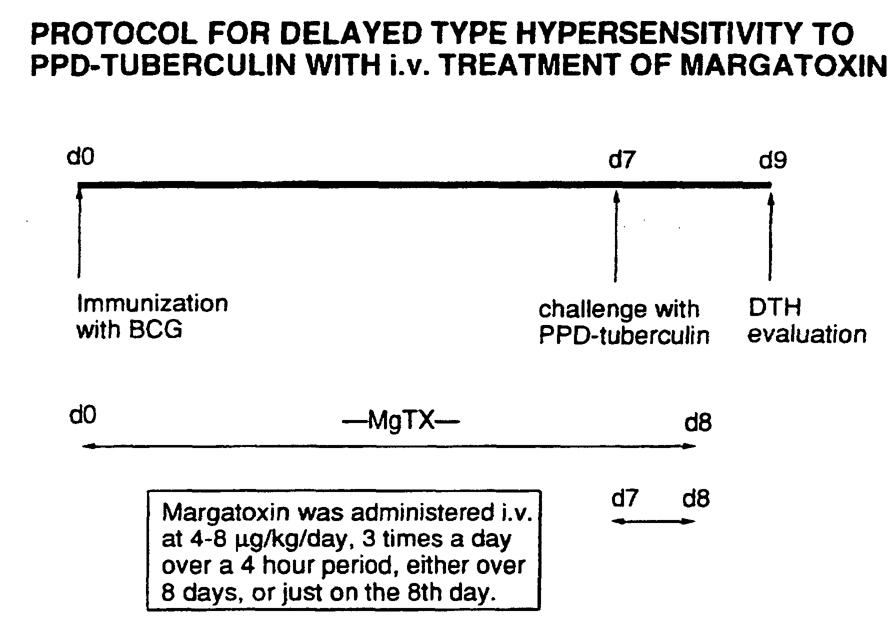

Initially, MgTX was delivered from the day of Bacillus-Calmette-Guerin (BCG) immunization to the day after tuberculin challenge and 50-80% suppression of the response was observed. However, when experiments were carried out to suppress the response by delivering MgTX only on the day of challenge, the same effect was observed.

Whole heparinized blood was collected, diluted with equal volume of RPMI (GIBCO) and centrifuged over ficoll-hypaque (LSM, Organo Technika Co., Durham, NC). The mononuclear cell fraction was isolated and adjusted to a concentration of about 2 x 10⁶/ml. Then 100 µl of the diluted mononuclear cell fraction was added to each well of a flat-bottom 96-well microtiter plate. 100 µl containing PMA (final concentration of 0.1-0.5 ng/ml) and ionomycin (final concentration of 200 ng/ml) was added. In some assays, where the suppressive effect of margatoxin was tested *in vitro,* 50 µl of media or margatoxin (final conc. 50-200 nM) was added. The cells were then cultured for 2 days at 37°C and 7% CO₂. [³H]Thymidine was added for the last 24 h. The cells were harvested and thymidine incorporation was counted by the LKB beta plate system.

Prior to conducting the pharmokinetic studies, we have spiked whole blood with Margatoxin and observed significant suppression of the proliferative response to PMA/ION, tested under the conditions described herein. In the pharmokinetic studies, blood samples were taken at the designated times and 1 ml of heparinized blood was spun and the plasma levels of margatoxin were determined. Two ml of blood was separated over ficoll-hypaque, and the MNC were tested for PMA/ION induction, as described above.

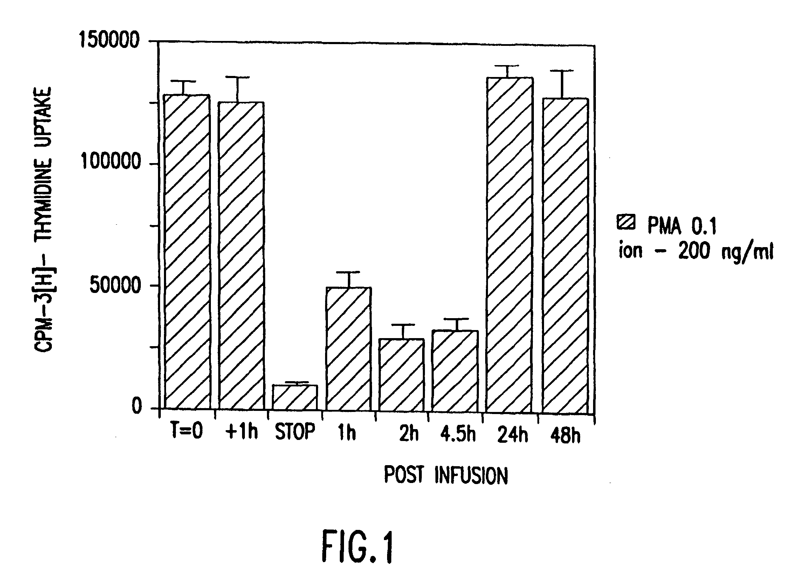

A representative experiment is shown in Figure 1. A Yucatan mini-pig received 25 µg of margatoxin in 30 ml of vehicle (5% autologous serum in 1:1 saline to PBS solution) over 2.5 h. There was no significant rise in blood pressure or heart rate during infusion. Plasma level in this study peaked at 600 pM, at the end of infusion, and dropped to 100 pM at 4.5 h post-infusion, with half-life of about 2 h. Margatoxin was undetectable in the plasma in 24 h. Plasma level of MgTX was determined in a competition binding assay, by its inhibition of ¹²⁵I-labeled MgTX binding to Kv1.3 channels present in isolated plasma membranes. Unresponsiveness to PMA/ION was also maximal at the end of infusion, and sustained for 4.5 h post-infusion. Similar pharmokinetic study was performed six times, with good correlations between high plasma levels of margatoxin and the lower response to PMA/ION. Yucatan mini-pigs receiving vehicle had no significant fluctuation of responses to PMA/ION. Therefore, this *ex vivo* test provides an assessment of the *in vivo* biological effect of margatoxin and can also be used to study the immunosuppressant effects of other ion channel blockers.

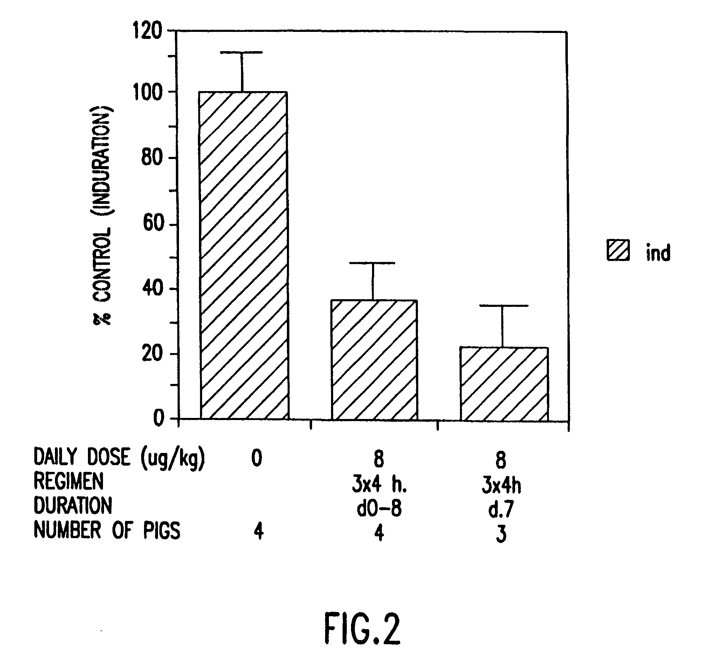

A representative graph of the inhibition by Margatoxin of the delayed type hypersensitivity response to PPD-tuberculin is shown in Figure 2. The Yucatan mini-pig was immunized with BCG 8 days (-8 d) prior to the challenge with PPD-tuberculin (PPD). MgTX was given on the eight days prior to the challenge or on the day of the challenge (0 d). PPD-tuberculin was injected intradermally at 4-6 spots on the flank of the pig. As control, PBS, the diluent for PPD was injected at 4-6 sites. Measurement of the antigen response, specifically the redness and induration was recorded for both the test and conrol injection sites at about 44 to 48 hours after the challenge.

## Claims

1. A method for analyzing an *ex vivo* effect of a Kv1.3 ion channel blocker administered *in vivo* to a test pig wherein the analysis comprises the steps of:
(a) non-therapeutically immunizing the test pig and a control pig with an antigen;
(b) measuring the immune response of the test and the control pig;
(c) non-therapeutically administering the Kv1.3 ion channel blocker to the test pig;
(d) administering the vehicle to the control pig;
(e) measuring the immune response of the test and the control pig;
(f) challenging the test and the control pig with the antigen;
(g) measuring the immune response of the test pig relative to the immune response of the control pig; and
(h) measuring the antigen response of the test and the control pig relative to the antigen response of the control pig.

2. The method according to Claim 1, wherein the analysis comprises the steps of:
(a) diluting whole heparinized blood collected from the pig with an equal volume of RPMI;
(b) centrifuging the RPMI-diluted blood solution over ficoll-hypaque;
(c) isolating the mononuclear cell fraction from the centrifuged RPMI-diluted blood solution;
(d) adjusting the concentration of the mononuclear cell fraction to about 2-million cells per mL;
(e) reacting 100µL of a solution of PMA at 0.1 to 0.5 ng/mL and ionomycin at 200 ng/mL to the microtiter wells with 100 µL of the mononuclear cells;
(f) incubating the PMA-ionomycin reaction for about 24 hours;
(g) incubating the incubated PMA-ionomycin reaction for an additional 24 hours with ³H-thymidine;
(h) harvesting the mononuclear cells after incubation with ³H-thymidine;
(i) counting the ³H-thymidine incorporation into the mononuclear cells.

3. A method for analyzing an *ex vivo* effect of a Kv1.3 ion channel blocker administered *in vivo* to a test pig wherein the analysis comprises the steps of:
(a) non-therapeutically immunizing the test pig and a control pig with an antigen;
(b) measuring the immune response of the test and the control pig comprising the steps of:
(i) diluting the test and control samples of whole heparinized blood collected from the test and control pigs with an equal volume of RPMI;
(ii) centrifuging the test and control samples of RPMI-diluted blood solution over ficoll-hypaque;
(iii) isolating the test and control samples of the mononuclear cell fraction from the centrifuged RPMI-diluted blood solution;
(iv) adjusting the concentration of the test and control samples of the mononuclear cell fraction to about 2-million cells per mL;
(v) reacting 100µL of a solution of PMA at 0.1 to 0.5 ng/mL and ionomycin at 200 ng/mL to the microtiter wells with 100 µL of the test and control samples of the mononuclear cells;
(vi) incubating the test and control samples of the PMA-ionomycin reaction for about 24 hours;
(vii) incubating the test and control samples of the incubated PMA-ionomycin reaction for an additional 24 hours with ³H -thymidine;
(viii) harvesting the test and control samples of the mononuclear cells after incubation with ³H-thymidine;
(ix) counting the ³H-thymidine incorporation into the test and control samples of the mononuclear cells to determine the relative immune response;
(c) non-therapeutically administering into a vein of the test pig a solution of the Kv1.3 ion channel blocker at a daily dose of 2 µg/kg to 100 mg/kg using an infusion pump dosing at a rate of 2.0 to 12 mL/hour;
(d) measuring the immune response of the dosed-test-pig as recited above in step (b);
(e) challenging the test and control pigs with the antigen;
(f) measuring the immune response of the antigen-challenged-test-pig and control pig as recited above in step (b);
(g) measuring the antigen response of the test pig and the control pig.

4. The method according to any one of Claims 1 to 3, wherein for the analysis the pig model used is a Yucatan mini-pig or a Yucatan micro-pig.

5. The method according to Claim 4, wherein for the analysis the pig model used is a Yucatan mini-pig.

6. The method according to any one of Claims 1 to 5, wherein for the analysis the antigen used to immunize the pig is Bacillus-Calmette-Guerin and the antigen used to challenge the pig is PPD-tuberculin.

7. The method according to any one of Claims 1 to 6, wherein for the analysis the Kv1.3 ion channel blocker is administered orally and the daily dose of the ion channel blocker is 0.1 mg/kg to 100 mg/kg.

8. The method according to any one of Claims 1 to 6, wherein for the analysis the Kv1.3 ion channel blocker is administered intravenously and the daily dose of the ion channel blocker is 2 µg/kg to 15 mg/kg.

## Patentansprüche

1. Verfahren zur Analyse einer *ex vivo*-Wirkung eines Kv1.3-lonenkanalblockers, welcher *in vivo* einem Testschwein verabreicht wird, wobei die Analyse die folgenden Schritte umfasst:
(a) Nicht-therapeutisches Immunisieren des Testschweins und eines Kontrollschweins mit einem Antigen;
(b) Messen der Immunreaktion des Testschweins und des Kontrollschweins;
(c) Nicht-therapeutisches Verabreichen des Kv1.3-Ionenkanalblockers an das Testschwein;
(d) Verabreichen des Vehikels an das Kontrollschwein;
(e) Messen der immunreaktion des Testschweins und des Kontrollschweins;
(f) Herausfordern des Testschweins und des Kontrollschweins mit dem Antigen;
(g) Messen der Immunreaktion des Testschweins im Vergleich zur Immunreaktion des Kontrollschweins, und
(h) Messen der Antigenreaktion des Testschweins und des Kontrollschweins im Vergleich zur Antigenreaktion des Kontrollschweins.

2. Verfahren nach Anspruch 1, wobei die Analyse die folgenden Schritte umfasst:
(a) Verdünnen von heparinisiertem Vollblut, das von dem Schwein gewonnen wurde, mit einem gleichen Volumen an RPMI;
(b) Zentrifugieren der RPMI-verdünnten Blutlösung über Ficoll-Hypaque;
(c) Isolieren der Fraktion mononukleärer Zellen aus der zentrifugierten RPMI-verdünnten Blutlösung;
(d) Einstellen der Konzentration der Fraktion mononukleärer Zellen auf etwa 2 Millionen Zellen pro mL;
(e) Umsetzen von 100 µL einer Lösung von PMA mit 0,1 bis 0,5 ng/mL und lonomycin mit 200 ng/mL auf den Mikrotitermulden mit 100 µL der mononukleären Zellen;
(f) Inkubieren der PMA-lonomycin-Reaktion für etwa 24 Stunden;
(g) Inkubieren der inkubierten PMA-lonomycin-Reaktion für weitere 24 Stunden mit ³H-Thymidin;
(h) Ernten der mononukleären Zellen nach Inkubation mit ³H-Thymidin;
(i) Zählen der ³H-Thymidin-Inkorporation in die mononukleären Zellen.

3. Verfahren zur Analyse einer *ex vivo*-Wirkung eines Kv1.3-Ionenkanalblockers, welcher *in vivo* einem Testschwein verabreicht wird, wobei die Analyse die folgenden Schritte umfasst:
(a) Nicht-therapeutisches Immunisieren des Testschweins und eines Kontrollschweins mit einem Antigen;
(b) Messen der Immunreaktion des Testschweins und des Kontrollschweins, umfassend die Schritte:
(i) Verdünnen der Test- und Kontrollproben von heparinisiertem Vollblut, das von den Test- und Kontrollschweinen gewonnen wurde, mit einem gleichen Volumen an RPMI;
(ii) Zentrifugieren der Test- und Kontrollproben von RPMI-verdünnter Blutlösung über Ficoll-Hypaque;
(iii) Isolieren der Test- und Kontrollproben der Fraktion mononukleärer Zellen aus der zentrifugierten RPMI-verdünnten Blutlösung;
(iv) Einstellen der Konzentration der Test- und Kontrollproben der Fraktion mononukleärer Zellen auf etwa 2 Millionen Zellen pro mL;
(v) Umsetzen von 100 µL einer Lösung von PMA mit 0,1 bis 0,5 ng/mL und lonomycin mit 200 ng/mL auf den Mikrotiter-Mulden mit 100 µL der Test- und Kontrollproben der mononukleären Zellen;
(vi) Inkubieren der Test- und Kontrollproben der PMA-lonomycin-Reaktion für etwa 24 Stunden;
(vii) Inkubieren der Test- und Kontrollproben der inkubierten PMA-lonomycin-Reaktion für weitere 24 Stunden mit ³H-Thymidin;
(viii) Ernten der Test- und Kontrollproben der mononukleären Zellen nach Inkubation mit ³H-Thymidin;
(ix) Zählen der ³H-Thymidin-Inkorporation in die Test- und Kontrollproben der mononukleären Zellen, um die relative lmmunreaktion zu bestimmen;
(c) Nicht-therapeutisches Verabreichen einer Lösung des Kv1.3-lonenkanalblockers in eine Vene des Testschweins mit einer täglichen Dosis von 2 µg/kg bis 100 mg/kg unter Verwendung einer Infusionspumpe mit einer Dosisrate von 2,0 bis 12 mL/Stunde;
(d) Messen der Immunreaktion des dosierten Testschweins wie oben in Schritt (b) angegeben;
(e) Herausfordern der Test- und Kontrollschweine mit dem Antigen;
(f) Messen der imniunreaktion des mit Antigen herausgeforderten Testschweins und Kontrollschweins wie oben in Schritt (b) angegeben;
(g) Messen der Antigenreaktion des Testschweins und des Kontrollschweins.

4. Verfahren nach irgendeinem der Ansprüche 1 bis 3, wobei für die Analyse das verwendete Schweinemodell ein Yucatan-Minischwein oder ein Yucatan-Mikroschwein ist.

5. Verfahren nach Anspruch 4, wobei für die Analyse das verwendete Schweinemodell ein Yucatan-Minischwein ist.

6. Verfahren nach irgendeinem der Ansprüche 1 bis 5, wobei für die Analyse das zur Immunisierung des Schweins verwendete Antigen Bacillus-Calmette-Guerin ist und das zur Herausforderung des Schweins verwendete Antigen PPD-Tuberculin ist.

7. Verfahren nach irgendeinem der Ansprüche 1 bis 6, wobei für die Analyse der Kv1.3-Ionenkanalblocker oral verabreicht wird und die tägliche Dosis des lonenkanalblockers 0,1 mg/kg bis 100 mg/kg beträgt.

8. Verfahren nach irgendeinem der Ansprüche 1 bis 6, wobei für die Analyse der Kv1.3-lonenkanalblocker intravenös verabreicht wird und die tägliche Dosis des lonenkanalblockers 2 µg/kg bis 15 mg/kg beträgt.

## Revendications

1. Procédé pour analyser un effet ex vivo d'un agent bloquant les canaux ioniques Kv1.3 administré *in vivo* à un cochon à tester, dans lequel l'analyse comprend les étapes consistant :
(a) à immuniser de manière non thérapeutique le cochon à tester et un cochon témoin par un antigène ;
(b) à mesurer la réponse immunitaire du cochon à tester et du cochon témoin ;
(c) à administrer de façon non thérapeutique l'agent bloquant les canaux ioniques Kv1.3 au cochon à tester ;
(d) à administrer le véhicule au cochon témoin ;
(e) à mesurer la réponse immunitaire du cochon à tester et du cochon témoin ;
(f) à stimuler le cochon à tester et le cochon témoin par l'antigène ;
(g) à mesurer la réponse immunitaire du cochon à tester par rapport à la réponse immunitaire du cochon témoin ; et
(h) à mesurer la réponse à l'antigène du cochon à tester et du cochon témoin par rapport à la réponse à l'antigène du cochon témoin.

2. Procédé selon la revendication 1, dans lequel l'analyse comprend les étapes consistant :
(a) à diluer du sang entier hépariné collecté à partir du cochon par un volume égal de RPMI ;
(b) à centrifuger la solution de sang diluée par du RPMI sur Ficoll-Hypaque ;
(c) à isoler la fraction de cellules mononucléaires de la solution de sang diluée par du RPMI centrifugée ;
(d) à ajuster la concentration de la fraction de cellules mononucléaires à environ 2 millions de cellules par ml ;
(e) à faire réagir 100 µl d'une solution de PMA entre 0,1 et 0,5 ng/ml et d'ionomycine à 200 ng/ml dans des puits de microtitrage avec 100 µl des cellules mononucléaires ;
(f) à incuber le mélange réactionnel PMA-ionomycine pendant environ 24 heures ;
(g) à incuber le mélange réactionnel PMA-ionomycine incubé pendant encore 24 heures avec de la ³H-thymidine ;
(h) à récolter les cellules mononucléaires après incubation avec de ³H-thymidine ;
(i) à compter l'incorporation de ³H-thymidine dans les cellules mononucléaires.

3. Procédé pour analyser un effet *ex vivo* d'un agent bloquant les canaux ioniques Kv1.3 administré *in vivo* à un cochon à tester, dans lequel l'analyse comprend les étapes consistant :
(a) à immuniser de manière non thérapeutique le cochon à tester et un cochon témoin par un antigène ;
(b) à mesurer la réponse immunitaire du cochon à tester et du cochon témoin, comprenant les étapes consistant :
(i) à diluer les échantillons à tester et témoin de sang entier hépariné collecté à partir du cochon à tester et du cochon témoin par un volume égal de RPMI :
(ii) à centrifuger les échantillons à tester et témoin de solution de sang diluée par du RPMI sur Ficoll-Hypaque ;
(iii) à isoler les échantillons à tester et témoin de fraction de cellules mononucléaires de la solution de sang diluée par du RPMI centrifugée ;
(iv) à ajuster la concentration des échantillons à tester et témoin de fraction de cellules mononucléaires à environ 2 millions de cellules par ml ;
(v) à faire réagir 100 µl d'une solution de PMA entre 0,1 et 0,5 ng/ml et d'ionomycine à 200 ng/ml dans des puits de microtitrage avec 100 µl des échantillons à tester et témoin de de cellules mononucléaires ;
(vi) à incuber les échantillons à tester et témoin de mélange réactionnel PMA-ionomycine pendant environ 24 heures ;
(vii) à incuber les échantillons à tester et témoin de mélange réactionnel PMA-ionomycine incubé pendant encore 24 heures avec de la ³H-thymidine ;
(viii) à récolter les échantillons à tester et témoin de cellules mononucléaires après incubation avec de ³H-thymidine ;
(ix) à compter l'incorporation de ³H-thymidine dans les échantillons à tester et témoin de cellules mononucléaires pour déterminer la réponse immunitaire relative ;
(c) à administrer de façon non thérapeutique dans une veine du cochon à tester une solution d'agent bloquant les canaux ioniques Kv1.3 à une dose quotidienne de 2 µg/kg à 100 mg/kg en utilisant une pompe à perfusion injectant à un débit de 2,0 à 12 ml/heure ;
(d) à mesurer la réponse immunitaire du cochon tester ayant reçu la dose comme il est décrit ci-dessus dans l'étape (b) ;
(e) à stimuler le cochon à tester et le cochon témoin par l'antigène ;
(f) à mesurer la réponse immunitaire du cochon à tester et du cochon témoin stimulés par l'antigène comme il est décrit ci-dessus dans l'étape (b) ; et
(g) à mesurer la réponse à l'antigène du cochon à tester et du cochon témoin.

4. Procédé selon l'une quelconque des revendications 1 à 3, dans lequel, pour l'analyse, le modèle de cochon utilisé est un mini-cochon du Yucatan ou un micro-cochon du Yucatan.

5. Procédé selon la revendication 4, dans lequel, pour l'analyse, le modèle de cochon utilisé est un minicochon du Yucatan.

6. Procédé selon l'une quelconques des revendications 1 à 5, dans lequel pour l'analyse, l'antigène utilisé pour immuniser le cochon est un bacille Calmette-Guérin et l'antigène utilisé pour stimuler le cochon est une tuberculine PPD (tuberculine purifiée).

7. Procédé selon l'une quelconque des revendications 1 à 6, dans lequel, pour l'analyse, l'agent bloquant les canaux ioniques Kv1.3 est administré par voie orale et la dose quotidienne de l'agent bloquant les canaux ioniques est de 0,1 mg/kg à 100 mg/kg.

8. Procédé selon l'une quelconque des revendications 1 à 6, dans lequel, pour l'analyse, l'agent bloquant les canaux ioniques Kv1.3 est administré par voie intraveineuse et la dose quotidienne de l'agent bloquant les canaux calciques est de 2 µg/kg à 15 mg/kg.
